# EUROPEAN PATENT SPECIFICATION

(11) **EP 0 754 029 B1**
(45) Date of publication and mention of the grant of the patent: **11.12.2002**
(21) Application number: 95914459.3
(22) Date of filing: 06.04.1995
(51) Int. Cl.: A61K 7/32, C08F 8/44

(54) **COMPOSITIONS CONTAINING FILM FORMING ANTIPERSPIRANT POLYMERS**
ZUSAMMENSETZUNGEN ENTHALTEND FILMBILDENDE SCHWEISSHEMMENDE POLYMERE
COMPOSITIONS COMPRENANT DES POLYMERES FILMOGENES ANTISUDORAUX

(30) Priority: 07.04.1994 GB 9406854
(43) Date of publication of application: 22.01.1997
(73) Proprietor: THE GILLETTE COMPANY, Boston, MA 02199-4099 (US)
(72) Inventor: CAUSTON, Brian Edward, Berkshire RG7 4LT (GB); BAINES, Frederick Charles, Bedfordshire LU6 2ED (GB)
(74) Representative: Ebner von Eschenbach, Jennifer
(86) International application number: GB9500800
(87) International publication number: WO95027473

(56) References cited:
- EP-A- 0 141 269
- EP-A- 0 162 388
- EP-A- 0 300 490
- FR-A- 2 250 516
- US-A- 3 966 902
- US-A- 4 057 533
- US-A- 4 883 608
- US-A- 5 209 922
- PATENT ABSTRACTS OF JAPAN vol. 13, no. 532 (C-659) (3880) 28 November 1989 & JP,A,01 216 916 (DAICEL CHEM IND LTD) 30 August 1989

## Description

This invention relates to the non-therapeutic use, as antiperspirants, of certain film-forming polymers.

It has been known for many years to use basic aluminium chloride (ACH) as an antiperspirant material, and products containing it are commonly available for example as aerosols, sticks, roll-ons, gels and creams. Whilst ACH is a very effective antiperspirant, it has some drawbacks and there is concern generally as to the desirability of using aluminium-containing materials for this purpose.

WO 93/24105 describes topical antiperspirant compositions consisting essentially of an effective antiperspirant amount of a non-toxic water-insoluble occlusive film-forming polymer. Among the preferred polymers are alkyl olefinic acid amide/olefinic acid or ester copolymers, alone or in combination with a PVP-linear alpha-olefin copolymer or other water-repellent polymer. These compositions are said to function as antiperspirants by the formation of a water-insoluble occlusive film on the skin, which reduces under-arm perspiration. They are described for use with ACH or alone.

There have been many proposals in the past to apply film-forming polymers to the skin for various purposes. The achievement of good antiperspirancy in this way has, however, proved very difficult. Not only is the quality of antiperspirancy difficult to find, but it is also difficult to overcome the important problem of providing adequate substantivity of the polymer towards the skin so that it remains in place in use.

We now describe some film-forming polymers which can provide excellent substantivity when applied topically to the skin, and additionally provide good antiperspirancy.

According to one aspect of the present invention, there is provided the non-therapeutic use, as an antiperspirant, of a film-forming polymer comprising units of the formulae: 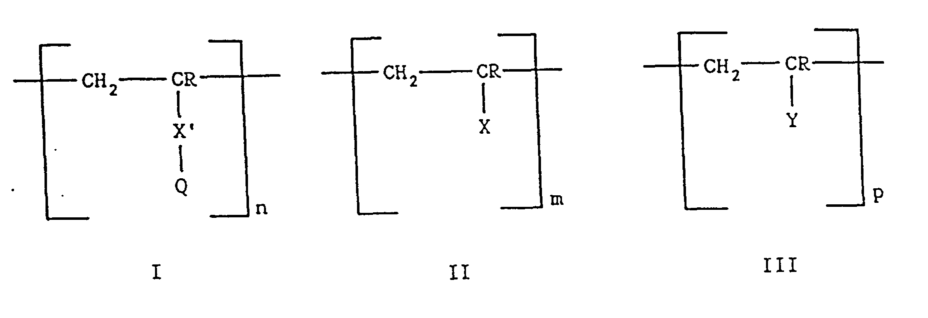 wherein:
X is a group which can be directly quaternised by reaction with an amine, or itself comprises a quaternisable nitrogen atom;
X'-Q is quaternised X, where Q is the quaternised nitrogen group and X' may be non-existent;
Q has at least one quaternised nitrogen atom having at least one substituent thereon which is hydrophobic and contains at least 8 carbon atoms;
Y is any atom or group which cannot be directly
   quaternised and does not comprise a quaternisable nitrogen atom;
R is hydrogen or alkyl (the alkyl preferably being C₁ to C₆):
m and p can each be 0 or an integer;
(n+m+p) is from 20 to 2000. preferably 200 to 1000;
n/(n+m+p)% is from 1% to 100% preferably 25% to 95%;
and wherein the polymer can contain two or more different units of formula I, formula II and/or formula III.

These polymers are useful as antiperspirants, and the invention also includes an antiperspirant composition which comprises 1% to 20% by weight of a film-forming polymer of the invention and a cosmetically acceptable non-aqueous carrier therefor which may be in admixture with a minor amount of water.

In the compositions of the invention, X is a group which either contains a quaternisable nitrogen atom, or which can be directly quaternised by reaction with an amine. The amine(s) can itself be in quaternised form if desired.

The compositions of the invention can contain a small amount (i.e. usually less than 5% by weight) of an aluminium antiperspirant, but in general the presence of aluminium antiperspirants reduces the efficacy of the compositions and we prefer the compositions to be substantially free from aluminium. The compositions can be formulated into aerosol, solid stick, roll-on, gel or cream formulations, for example.

Patent Abstracts of Japan, Vol. 13, No. 532 (C-659) [3880] and JP-A-1 216 916 disclose a composition containing a polymeric quaternary ammonium salt which is a reaction product of a homopolymer of (meth)acrylic acid and a quaternary ammonium salt. The composition is applied to the armpit to absorb perspiration and eliminate the odour emitted therefrom.

FR-A-2 250 516 describes non-aqueous aerosol antiperspirant compositions which comprise a moisture-absorbent organic polymer which is non-sticky when swollen with water. Polymers described as being suitable range from water-soluble polymers, such as natural starches and polyvinylalcohol, to water-insoluble polymers such as calcium sodium alginate, crosslinked polyacrylamides, crosslinked polyvinylpyrrolidone and quaternised polyvinyl pyridine crosslinked with di-vinyl benzene.

US-A-3 966 902 describes compositions comprising a polymer complex carrier and an active ingredient (e.g. pesticide, antiperspirant, etc.) entrapped therein. The polymer is formed at least in part from a monomer having hydrophilic functional groups and containing aluminum, zinc or zirconium metal bound in complex form.

EP-A-0 162 388 discloses particular quatemized vinylimidazole-ethylene glycol dimethacrylate copolymers that are said to be useful as bile acid sequestering agents and inhibitors of diarrhoea.

US-A-5 209 922 discloses an antifungal block copolymer of poly(vinyl lactam) and a quaternized amino alkyl acrylamide comonomer.

EP-A-0 300 490 describes the preparation of cationic polyvinyl alcohol by blending polyvinyl alcohol under high-shear conditions with water, a base, and a quaternizing agent containing a halohydrin radical and a quaternary ammonium group.

US-A-4 057 533 discloses a composition comprising quaternized cationic vinyllactamacrylamide copolymers useful as flocculants and filler retention aids in paper-making.

US-A-4 883 608 describes a polymeric chemical warfare agent decontamination composition comprising a polymeric quaternary pyridinium salt resulting from the reaction of a polyvinyl benzyl salt and a dialkylaminopyridine.

European patent specification no. 0141269 describes certain polyvinyl alcohol polymers having oxylinked pendant quaternary ammonium or tertiary amine groups, as aids in reducing moisture loss when applied to skin as conditioning lotions or ointments in cosmetic and pharmaceutical formulations. In general, moisture loss polymers of this type function by forming a thin film on the surface of the skin. This film reduces transepidermal loss of moisture. However, this is a different effect from the antiperspirancy function of ACH. An antiperspirant functions to stop or significantly reduce the aqueous discharge from sweat ducts, and this is two or more orders of magnitude greater than transepidermal moisture loss. Thus, film-forming polymers known for use in reducing transepidermal loss cannot be expected simultaneously to provide any significant antiperspirant effect. In EP-A-0141269, the polyvinyl alcohol derivatives are used in amounts from 0.5 - 5% by weight of antiperspirant compositions containing conventional amounts of ACH, one example being 4% of the polymer in a composition containing 57% Al Zr tetrachloro-hydrex-Gly Rezol 36G (Reheis). This is quite different from the compositions of the present invention where amounts of ACH greater than about 5% tend to cause a reduction of antiperspirant efficacy.

Further, the polyvinyl alcohol polymers described in EP-A-0141269 are soluble or dispersible in water so that they will function to reduce transepidermal loss and be compatible with conventional aqueous-based cosmetic and personal care products. In contrast, the polymers used in the present invention are hardly soluble or dispersible in water at all, and require an alcoholic or other non-aqueous solvent carrier. Indeed, dispersions are unstable in the presence of significant amounts of water.

In the polymers described herein, it is important that the quaternary nitrogen atoms have a hydrophobic substituent of relatively large size, i.e. at least C₈. The effect of this hydrophobic substituent is to cause the polymer to change shape in the presence of any water which will be present during use of the polymer as an antiperspirant. It is believed that the change of shape is such as to express the ionic groups and this significantly improves the substantivity towards human skin. The polymers of the invention thus have good substantivity and also show a marked antiperspirant effect.

A preferred substituent for the quaternary nitrogen group is a substituted or unsubstituted hydrocarbyl group containing from 8 to 25 carbon atoms. Possible substituents include, for example, halogen, amino, nitrate, hydroxyl or aryl substituents. More preferably, the hydrocarbyl group is a linear saturated group, most preferably an alkyl group, especially of 12 to 18 carbon atoms, e.g. dodecyl or stearyl. It is to be understood that by "antiperspirant" we mean a substance which when applied to the skin as an antiperspirant reduces wetness by at least 20%. (Federal Register, October 10th 1978 (43 FR 46694) and Federal Register, August 20th 1982 (162 FR 36492).)

Conventionally, antiperspirants are usually formulated as roll-ons, sticks, aerosols, gels or creams. For the manufacture of these formulations, it is preferred in general that the active antiperspirant be in solution. Alternatively, a dispersion can be used provided it is relatively stable. Since the film-forming polymers used in the present invention are generally not soluble in water, they are dissolved or dispersed in a non-aqueous cosmetically acceptable carrier. The amount of film-forming polymer is from 1% to 20% by weight, preferably from 6% to 10% by weight. Examples of suitable carriers include alcohols such as ethanol, glycols such as propylene glycol, dipropylene glycol, butylene glycol, triols such as glycerol, propylene carbonate and volatile silicones including, for example, cyclic silicones, linear silicones and low molecular weight dimethicones. Some water can be included provided that the composition remains as a solution or is a stable dispersion. The addition of too much water will destabilise the dispersions. The amount of water which can be tolerated varies from polymer to polymer and with the non-aqueous carrier, and may be as little as 1% up to amounts of 30% or more. It will, however, always be less than the amount of non-aqueous carrier, i.e. it is present in minor amount relative thereto. In any particular case, routine trials will indicate the limits. It is preferred that, in the final formulation, some water be present.

In the case of dispersions of the invention, it is preferred that the disperse phase be from 5 to 10 micrometres in size.

The precise choice of film-forming polymer is not critical. Among the preferred polymers are those wherein, in the units of formula I, X' is an alkylene carbonyl oxy group(-R₄.CO.O-), a carbonyl oxyalkylene group (-CO.O.R₄-), or an arylene or aralkylene group (e.g.-C₆H₄-R₄-). Preferably, X' has the formula -O.CO.CH₂-(the oxygen being attached to the -CR-group) and R is hydrogen or methyl; or X' is a carbonyloxyethyleneoxyethylene group (the carbon being attached to the -CR-group) and R is methyl; or X' is a benzyl group (the benzyl -CH₂ being attached to Q) and the R is hydrogen. These polymers can be variously derived from, for example, polyvinylalcohol, haloalkyl polystyrenes and polyhydroxyalkyl methacrylates. Halogens can be introduced into hydroxy side-chains using chloroacetate. and the halogen then quaternised.

Other preferred values for X' in the formula I units are:

-CO.O.R₄- -O.CO.R₄-

-CO-

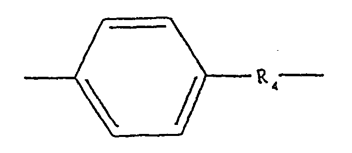

-CO.O.R₄-O.CO.R₄-

-R₄-O.CO.R₄₋

-CO.NH.CH₂O.COR₄₋

-CO.O.R₄.OR₄.OCOR₄₋

-CO.O.R₄.O.R₄₋

wherein each R₄ is independently an alkylene group of up to 6 carbon atoms, most preferably methylene or ethylene.

Film-forming polymers for use in the invention with values of X'-Q defined above can be made from many known polymers, including, for example, polyacrylamide, poly(haloalkylacrylate), poly(acryloyl chloride), chloromethylpolystyrene, poly(hydroxyalkyl methacrylate), polysaccharide. poly(allyl alcohol), poly(N-methylol acrylamide), poly(alkylacrylate), poly(alkylmethacrylate) and poly(glycidyl methacrylate). The side chains of these known polymers can be converted to the X'-Q side chains defined above in various ways, as will be clear to those skilled in the art. For example:
(a) the Mannich reaction can be used to quaternise the amido NH₂ group of polyacrylamide.
(b) those known polymers with halogen in the side chain can be quaternised directly by reaction with an amine.
(c) those polymers with a hydroxyl (or epoxide) group in the side chain can be converted to the chloroacetate (or an equivalent halocarboxylate) derivative which can then be quaternised.
(d) those polymers with a carboxyl ester group can be transesterified with, for example, a glycol and then treated as in (c). These procedures are merely examples.

The polymers can also, of course, be made from known polymers which have at least one quaternisable nitrogen atom present in a side chain. Such known polymers include, for example, polyvinyl pyridine, polyvinylimidazoline and polyvinyl imidazole. Polymers for use in the invention include:
(i) those which contain formula I units of formula 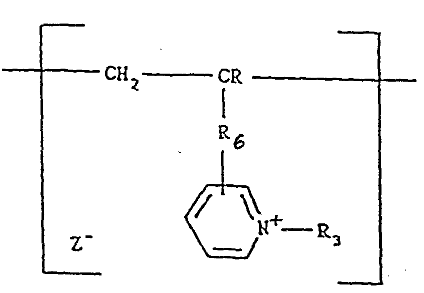 wherein R is hydrogen or an alkyl group, R₆ is a single bond or an alkylene group, R₃ is hydrophobic and contains at least 8 carbon atoms, and Z⁻ is an anion.
(ii) those wherein, in the units of formula I, X'-Q is selected from 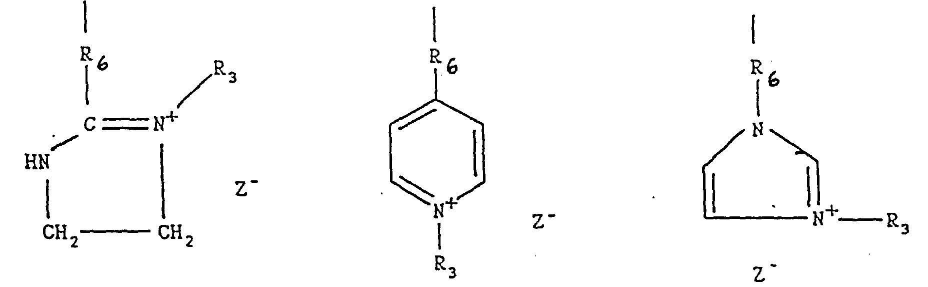 wherein R₆ is a single bond or an alkylene group of 1 to 6 carbon atoms, R₃ is hydrophobic and contains at least 8 carbon atoms. and Z⁻ is an anion.

Among the preferred polymers for use in the invention are those which comprise units of formula II wherein X is selected from
-Hal ; -R₄Hal ; -O.CO.R₄.Hal
CO.O.R₄.Hal ; -CO.Hal ; 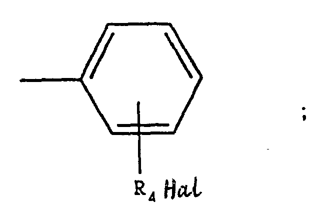 CO.O.R₄.OCOR₄Hal ;
CO.NH.O.CO.R₄Hal ; 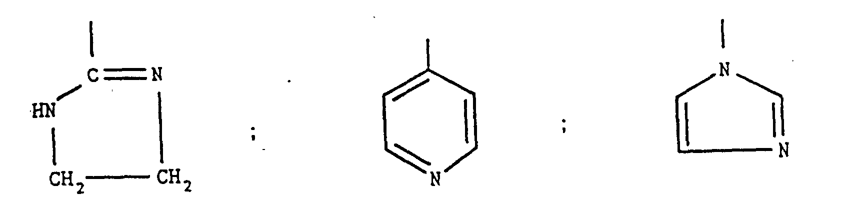 wherein R4 is an alkylene group of 1 to 6 carbon atoms, and Hal is a halogen, preferably chlorine or bromine.

R₄ is most preferably methylene or ethylene.

In the polymers for use in the invention, m and p can each independently be zero. Normally, neither m nor p will be zero. When p is not zero, preferred units of formula III include those wherein Y is selected from
-H; -OH; -CONH₂ ; -CO.O.R₄.OH ;
-R₄OH ; -CONHR₄OH ; 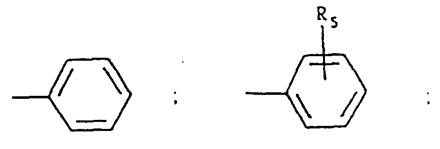 -CO.O.R₅ ; -CO.O.R₄.O.R₄.OH ;
wherein R₄ is an alkylene group of 1 to 6 carbon atoms, and R₅ is an alkyl group of 1 to 6 carbon atoms, and most preferably R4 is methylene or ethylene.

In the quaternised polymers for use in the invention, the quaternary nitrogen atoms have one long chain substituent i.e. one substituent having 8 or more, preferably 8 to 25. carbon atoms. Any hydrocarbyl group having from 8 or more carbon atoms can be used. Among the preferred such substituents are C₁₂ (dodecyl), C₁₆ (hexadecyl) and C₁₈ (stearyl) but other substituents may be employed, for example alkylaryl radicals. The preferred R₁ and R₂ substituents are hydrocarbyl, eg. alkyl groups, having from 1 to 8 carbon atoms. Methyl groups are preferred, but they may themselves carry substituents as desired. Among the possible substituents on the hydrocarbyl groups are halide, amino, nitro, hydroxyl or aryl, for example.

However, regardless of which particular values for the three substituents on the nitrogen are chosen, it is important that together they have a hydrophobicity to promote the substantivity of the polymer as previously described. The substantivity to skin can be impaired if the substituents are so bulky as to cause steric hindrance to the quaternary ammonium ion. Generally, two of the groups will be small relative to the main hydrophobic group of at least 8 carbon atoms.

The quaternised nitrogen atoms may be attached directly to the polymeric backbone but usually instead are attached to a side chain extending from the backbone. In order to promote the formation and stability of the quaternary ammonium groups, the side chain will preferably include some electron withdrawing atoms or groups. Thus, as described above, the side chains can be formed from halocarboxylates, eg. haloacetates, by substituting the quaternised nitrogen at the halogen position. Other halogenated side chains may also be employed to make the quaternised compounds, for example p-chloromethylphenyl units.

The side chain can also be a tertiary aromatic amine, for example 2-pyridyl or 4-pyridyl, or an aliphatic amine, for example dialkylaminoethyl ester and the quaternary formed by using haloalkyls.

The side chain can be any alcohol or ester, the quaternary being added either by the use of base or Lewis acid catalysis of a ring opening addition by a quaternised epoxy or transesterification of a trialkyl oxy-ammonium halide catalysed by a transition metal catalyst.

The nature of the carbon chain backbone of the polymer is not critical provided that it does not contain substituents antagonistic to the intended use of the polyquat. One highly preferred material is polyvinyl alcohol to which side chains can be attached via the pendant OH groups.

In the film-forming polymers for use in the invention, Q is preferably selected from:
(a) -N⁺R₁R₂R₃Z⁻ where R₁ and R₂ are the same or different and are each a C₁ to C₈ substituted or unsubstituted hydrocarbyl group, R₃ is a C₈ to C₂₅ substituted or unsubstituted hydrocarbyl group, and Z⁻ is an anion, the possible substituents being those described above;
(b) 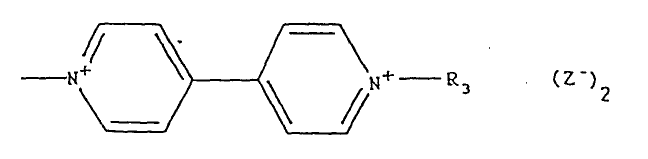 where R₃ and each Z- are independently as defined in (a) above;
(c) 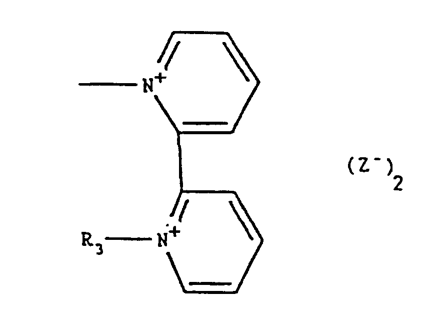 where R₃ and each Z⁻ are independently as defined in (a) above;
(d) 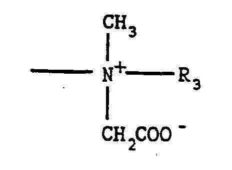 where R₃ is as defined in (a) above;
(e) 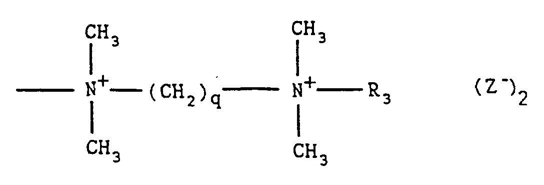 where R₃ and each Z⁻ are independently as defined
   in (a) above and q is from 2 to 10.

Preferably, R₁ and R₂ are both alkyl groups. R₃ is preferably a linear saturated hydrocarbyl group, preferably C₁₂ to C₁₈. In the above formulae for Q, and in all occurrences in the specification and claims, Z⁻ is preferably a halide ion, most preferably chlorine or bromine.

In the diquaternary (e) above, q is preferably 2,3 or 4.

The film-forming polymers used in the present invention can have a very high antiperspirant efficacy. (Efficacy was measured using a standard forearm sweat reduction test and the standard FDA axilla sweat reduction test (Federal Register August 20th 1982 (162 FR 36492.) For example, we have found that a concentration as low as 3% by weight in an aqueous alcohol (30% water) dispersion can be more efficacious than a conventional 20% ACH solution. The efficacy does vary among the polymers. In general, the efficacy increases with increasing chain length of the quaternary nitrogen substituents. Efficacy also tends to increase with increasing water-content of the compositions.

In the quaternised polymers for use in the invention, it is not essential that every side chain (or group pendant from the main backbone chain) be quaternised. With increasing quaternisation, efficacy tends to rise but very useful and adequate antiperspirancy can be achieved with relatively low quaternisation. Quaternisation can be as low as 1%, but we prefer from about 25% up to 95% or more, and more preferably at least 75%, most especially at least 85%.

The quaternised polymeric materials can be made in a variety of ways as previously described, depending on their precise constitution. Usually, however, a polymeric material with a suitably reactive side chain is reacted with a tertiary amine. For example, poly(vinylchloroacetate) can be reacted with stearyldimethylamine to make the antiperspirant polyquat stearyldimethylamine quaternised poly(vinylchloroacetate). The poly(vinylchloroacetate) can be made by reacting polyvinyl alcohol with chloroacetyl chloride. Those skilled in the art will know of these types of reactions and no further teaching in connection therewith will be given herein.

As stated above, the antiperspirant efficacy of the polyquats is improved by the presence of water. It is preferred, therefore, to use them in formulations where water can be present. Accordingly, we prefer to use them in roll-ons, creams, gels and stick formulations, rather than in aerosols.

The molecular weights of the polymers for use in the invention can vary widely, but we prefer to use polymers in which (m+n+p) is from 20 to 2000 units, most preferably 200 to 1000 units. In the case of a 25% degree of substitution, 25% of the units in the polymer will be of formula I above. At very high degrees of substitution e.g. 95%, virtually all the polymer units are those of formula I.

The polymers can contain two or more different formula I groups in the same polymer, i.e. mixed polyquats. These polymers are usually made by using two different amines in the quaternisation procedure. They are especially useful in providing a bactericidal effect.

The polymers for use in the invention can be homopolymers or copolymers made from two or more monomers. It can be advantageous to use copolymers in order to provide more closely the qualities desired in the film-forming polymer. For example, poly(vinylalcohol) polymers can be made softer by copolymerising the vinyl alcohol with ethylene. In the resulting polymers, the units derived from the ethylene are formula III units in which Y is hydrogen.

In order to make a conventional antiperspirant formulation using the film-forming antiperspirant polymers described herein, a composition of the invention is mixed with other components of the formulation. The various ways in which this is done will be clear to those skilled in the art. For example, sticks can be formed from a soap gel, a wax, or a dibenzylidine sorbitol product. No detailed description thereof will be given.

In order that the invention may be more fully understood, the following Examples are given by way of illustration only.

### EXAMPLE 1

### Preparation of chloroacetate derivatives of hydroxy polymers (not with scope of appended claims)

To the appropriate hydroxy polymer (0.1 mol) were added chloroacetyl chloride (0.3 mol) and water (a few drops). The heterogeneous mixture obtained was stirred at room temperature under anhydrous conditions for 4h. The viscous homogeneous mixture formed was diluted with ethyl acetate. The polymer was precipitated into an excess of ethanol, filtered and dried under vacuum to give a light brown powder. Yield 75%.

The reaction was performed on poly(vinylalcohol) samples of molecular weight 9,000, 14,000, 22,000 and 49,000, with degrees of polymerisation (DP) of (approximately) 200, 300, 500 and 1100, respectively, and degrees of hydrolysis of 98.4%, 88% and 80%, respectively. The degree of hydrolysis indicates the extent of conversion of polyvinylalcohol to polyvinylacetate.

### EXAMPLE 2

### Quaternisation of poly(vinvlchloroacetate) with stearyl dimethylamine (SDMA)

The SDMA quaternary was prepared by reacting poly(vinylchloroacetate) with stearyl dimethylamine: 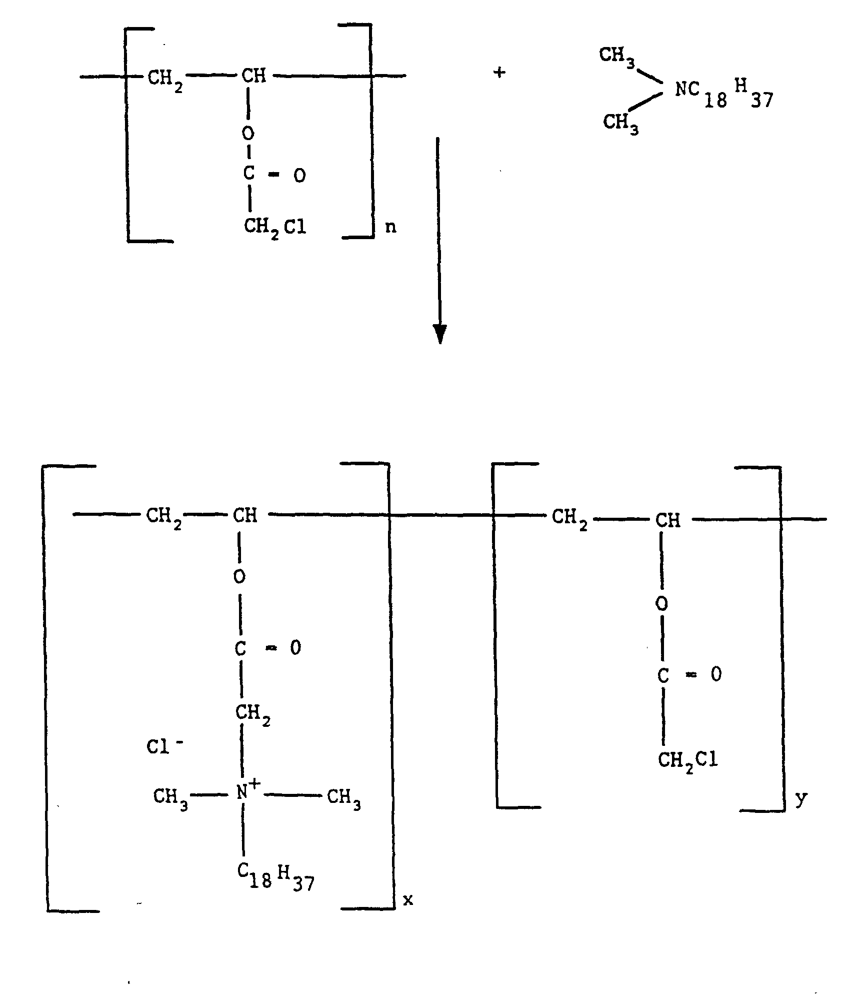 where n = x + y. The poly(vinylchloroacetate) was derived from 9000 molecular weight poly(vinylalcohol) as described in Example 1. This poly(vinylchloroacetate) (0.1 mol) was dissolved in ethyl acetate (150ml). To this solution was added the required amount of stearyldimethylamine. This reaction was refluxed for three hours. The quaternised polymer precipitated out. The excess ethyl acetate was decanted off and the product was triturated with ethyl acetate, filtered and dried in a vacuum oven at room temperature. When an equimolar amount of SDMA was added, the substitution was about 88% by nitrogen analysis. Elemental analysis indicated 88% quaternisation.

The ratio of poly(vinylchloroacetate) to SDMA was varied as follows: 1:0.5. 1:0.4, 1:0.3. 1:0.2 and 1:0.1 to provide a number of products of varying quaternisation. The same results were obtained when dimethylformamide was used as the solvent in place of ethyl acetate.

| | Theory (w/w%) | Found (w/w%) |
|---|---|---|
| Carbon | 69.0 | 64.5 |
| Hydrogen | 11.5 | 10.3 |
| Nitrogen | 3.4 | 3.0 |

### EXAMPLE 3

Example 2 was repeated but using dodecyldimethylamine instead of stearyl dimethylamine. Similar results were obtained.

### EXAMPLE 4

The efficacy of various solutions of the polyquats of Examples 2 and 3 was tested by routine methods and compared to conventional ACH solutions. The results showed that the stearyl quaternary was more efficacious than the dodecyl quaternary, and that efficacy increased with increasing degrees of quaternisation. In general, the efficacy was close to that of ACH. Substantivity to human skin was excellent. The results of the tests were as follows:

| Forearm Efficacy of Polyquat Formulations | | | | | |
|---|---|---|---|---|---|
| | Formulation % w/w | | | | Efficacy % |
| Polyvinylchloroacetate SDMA | Polymer | Ethanol | Volatile Silicone VS 344 | Water | |
| 1:1 | 8 | 62 | - | 30 | 70.6 |
| | 5 | 65 | - | 30 | 64.0 |
| | 3.5 | 66.5 | - | 30 | 54.0 |
| | 2 | 68 | - | 30 | 17.5 |
| | 8 | 72 | - | 20 | 47.8 |
| | 8 | 82 | - | 10 | 39.2 |
| | 8 | 62 | 30 | - | 45.9 |
| 1:0.5 | 8 | 62 | - | 30 | 55.3 |
| 1:0.25 | 8 | 62 | - | 30 | 43.8 |

| Polyvinylchloroacetate | | | | | |
|---|---|---|---|---|---|
| DDMA | | | | | |
| 1:1 | 8 | 62 | - | 30 | 56.6 |
| 1:0.25 | 8 | 62 | - | 30 | 16.8 |

The polyvinylchloroacetate is derived from polyvinyl alcohol (DP 320)

| Standard FDA Hot Room Efficacy Test | | | | | |
|---|---|---|---|---|---|
| | Formulation % w/w | | | | Efficacy % |
| Polyvinylchloroacetate SDMA | Polymer | Ethanol | Volatile Silicone VS 344 | Water | |
| 1:1 | 8 | 62 | - | 30 | 28.4 |
| Polyvinylchloroacetate derived from polyvinylalcohol (88% hydrolysed DP200) | | | | | |

### EXAMPLE 5

### Quaternisation of the chloroacetate derivative of poly(2-hydroxyethyl methacrylate) with stearyl dimethylamine

The chloroacetate derivative of poly(2-hydroxyethyl methacrylate) (0.01 mol) was dissolved in dimethylformamide (100 ml). To the solution was added stearyl dimethyldiamine (0.012 mol) and this was left stirring at 60°C for 72h. The polymer was precipitated into an excess of ethyl acetate, triturated with fresh ethyl acetate (twice) and dried under vacuum to give a pale yellow powder. Yield 60%.

| Elemental analysis indicated 40% quaternisation. | | |
|---|---|---|
| | Theory (w/w%) | Found (w/w%) |
| Carbon | 64.8 | 61.2 |
| Hydrogen | 10.6 | 9.5 |
| Nitrogen | 5.4 | 2.2 |

### EXAMPLE 6

### Quaternisation of poly(4-vinylpyridine) with 1-bromohexadecane

To a viscous solution of poly(4-vinylpyridine). molecular weight 50000, approximate DP 475, (0.02 mol) in methanol (100 ml) was added 1-bromohexadecane (0.04 mol) and this was stirred under reflux for 5 days. The solvent was removed under vacuum. The polymer was purified by repeated dissolution in dichloromethane and precipitation into an excess of cold diethyl ether. The polymer was dried under vacuum to give a light-brown powder. Yield 85%. Halide analysis indicated 97% quaternisation. Analysis for bromine revealed 18.9% (19.5% expected).

### EXAMPLE 7

### Quaternisation of Dolv(vinylbenzylchloride) with stearyl dimethylamine

Poly(vinylbenzylchloride), molecular weight 55000, approximate DP 360, (2g) and stearyl dimethylamine (3.9g) were added to ethanol (25ml) and heated under reflux with stirring for 24h. The reaction mixture was precipitated into acetone (400 ml) and centrifuged. A white powder was recovered and dried under vacuum to constant weight (5g). Elemental analysis indicated 90% quaternisation.

| | Theory (w/w%) | Found (w/w%) |
|---|---|---|
| Carbon | 77.4 | 71.8 |
| Hydrogen | 11.6 | 10.6 |
| Chlorine | 7.9 | 7.3 |
| Nitrogen | 3.1 | 2.7 |

### EXAMPLE 8

### Co-quaternisation of poly(vinylchloroacetate) with stearyl dimethylamine and 4,4'-dipyridyl N-benzyl bromide

Firstly, monoquaternisation of 4,4'-dipyridyl with benzyl bromide was effected as follows. Benzyl bromide (0.05 mol) was added dropwise to a solution of 4,4'-dipyridyl (0.065 mol) in dry acetone (100 ml) and the mixture was refluxed for 4h. The solid formed was filtered and crystallised from diethyl ether:ethanol to give the pure product. Yield 60%.

| | Theory (w/w%) | Found (w/w%) |
|---|---|---|
| Carbon | 62.2 | 62.1 |
| Hydrogen | 4.9 | 4.7 |
| Nitrogen | 8.5 | 8.5 |

Then, the monoquaternary of 4,4'-dipyridyl (2 x 10⁻⁴ mol) was added to a solution of poly(vinylchloroacetate) (0.01 mol) in dimethylformamide (100 ml) and stirred at 50°C for 24h. (The poly(vinylchloroacetate) was made by the method of Example 1 from poly(vinylalcohol) of molecular weight 14000, approximate DP 300.) Stearyl dimethylamine (1 x 10⁻² mol) was then added and heating and stirring continued for a further 48h. The precipitate which formed was triturated with ethyl acetate (twice) and dried to give a white powder. Yield 75%.

### EXAMPLE 9

### Co-quaternisation of poly(vinylchloroacetate) with stearyl dimethylamine and N-octadecyl-N,N,N',N'-tetramethylethylenediamine bromide

Firstly, monoquaternisation of N,N,N',N'-tetramethylethylenediamine with 1-bromooctadecane was effected as follows. 1-bromooctadecane (0.03 mol) was added dropwise to a solution of N,N,N',N'-tetramethylethylenediamine (0.04 mol) in ethanol (200 ml). The mixture was refluxed for 24h and the solvent removed under vacuum to give a waxy material. Yield 70%.

The monoquaternary of N,N,N',N'-tetramethylethylenediamine (2 x 10⁻⁴ mol) was added to a solution of poly(vinylchloroacetate) (0.01 mol) in dimethylformamide (100 ml) and stirred at 50°C for 24h. (The poly(vinylchloroacetate) was made from poly(vinylalcohol) of molecular weight 14000, approximate DP 300, by the method of Example 1.) Stearyl dimethylamine (1 x 10⁻² mol) was then added and heating and stirring continued for a further 48h. The precipitate which formed was triturated with ethyl acetate (twice) and dried to give a white powder. Yield 75%.

### EXAMPLE 10

### Quaternisation of poly(vinylalcohol-co-ethylene) with SDMA

Poly(vinylalcohol-co-ethylene) was converted to the chloroacetate derivative by the process of Example 1.

The poly(vinylchloroacetate-co-ethylene) (0.04 mol, ethylene content 27 mol%) was dissolved in dimethylformamide (150 ml). Stearyl dimethyldiamine (0.035 mol) was added and the mixture stirred at 50°C for 72h. The solid precipitate which formed was triturated with fresh ethyl acetate (twice) and dried under vacuum to given an off-white powder. Yield 80%.

### EXAMPLE 11

### Quaternisation of poly(styrene-co-allvl alcohol) with stearyl dimethylamine

Poly(styrene-co-allyl alcohol) molecular weight 1600, DP approximately 20, was converted to the chloroacetate derivative by the process of Example 1.

The poly(styrene-co-allyl chloroacetate) (0.04 mol. styrene content 94 mol%) was dissolved in ethyl acetate (150 ml). Stearyl dimethyldiamine (3 x 10⁻³ mol) was added and the mixture stirred at 50°C for 72h. The solvent was removed under vacuum. The polymer was purified by repeated dissolution in dichloromethane and precipitation into an excess of cold diethyl ether. The polymer was dried under vacuum to give a pale yellow powder. Yield 75%.

### EXAMPLE 12

An example of a roll-on of the invention is:

| | % |
|---|---|
| Polyquat (Example 2) | 8.0 |
| Triclosan | 0.3 |
| Water | 1.0 |
| Cyclomethicone DC344 | 30.0 |
| Ethanol | 60.7 |

### EXAMPLE 13

An example of a stick product of the invention is:

| | % |
|---|---|
| Polyquat (Example 2) | 8.00 |
| Ethanol | 53.95 |
| Water | 16.45 |
| Stearyl alcohol | 14.00 |
| Castor oil | 3.00 |
| Talc | 2.20 |
| Silica | 1.40 |
| PEG | 1.00 |

### EXAMPLE 14

An example of an aerosol of the invention is:

| | % |
|---|---|
| Polyvinylchloroacetate/DDMA (1:1) | 4 |
| | |
| Ethanol | 74.5 |
| Triethyl citrate | 1.5 |
| CAP 30 | 20 |

## Claims

1. The non-therapeutic use, as an antiperspirant, of a solution or dispersion of a film-forming polymer comprising units of the formula: 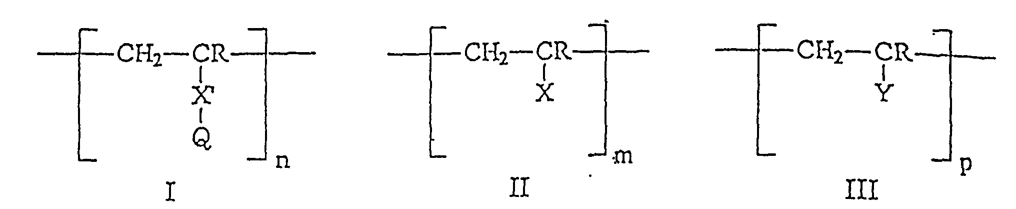 wherein:
X is a group which can be directly quaternised by reaction with an amine, or itself comprises a quaternisable nitrogen atom;
X'-Q is quaternised X, where Q is the quatemised nitrogen group and X' may be non-existent;
Q has at least one quaternised nitrogen atom having at least one substituent thereon which is hydrophobic and contains at least 8 carbon atoms;
Y is any atom or group which cannot be directly quaternised and does not itself comprise a quaternisable nitrogen atom;
R is hydrogen or alkyl;
m and p can independently be 0 or an integer;
(n+m+p) is from 20 to 2000;
n/(n+m+p) expressed as a percent is from 1% to 100%;
and wherein the polymer may optionally contain two or more different units of formula I, formula II and/or formula III.

2. A use according to claim 1, wherein n/(n+m+p)% is from 25% to 95%.

3. A use according to claim 1. wherein m and p are both integers and n/(n+m+p)% is from 1% to less than 100%.

4. A use according to claim 1, wherein X'-Q is selected from
-CO-O-R₄-Q -O-CO-R₄-Q -CO-Q
-R₄-CO-O-Q -C₆H₄-R₄-Q
-CO-O-R₄-O-CO-R₄-Q -R₄-O-CO-R₄-Q -CO-NH-CH₂O-CO-R₄-Q
-CO-O-R₄-O-R₄-Q -CO-O-R₄-O-R₄-O-CO-R₄-Q
wherein each R₄ is independently an alkylene group of 1 to 6 carbon atoms and a is selected from
(a) -N⁺R₁R₂R₃ Z⁻ 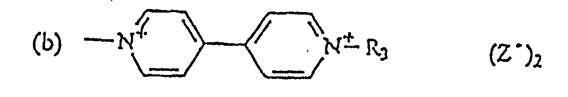 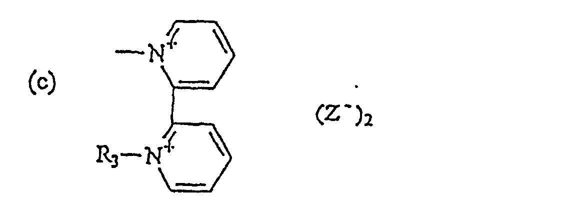 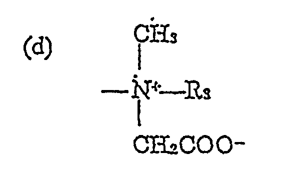 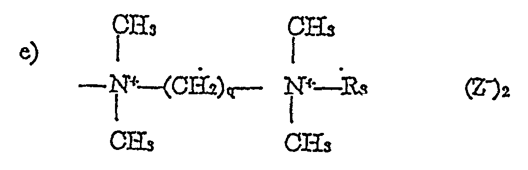
where R₁ and R₂ are the same or different and are each a C₁ to C₈ hydrocarbyl group unsubstituted or substituted with at least one halo, amino, nitro, hydroxyl or aryl group, R₃ is a C₈ to C₂₅ hydrocarbyl group unsubstituted or substituted with at least one halo, amino, nitro, hydroxyl or aryl group, q is an integer from 2 to 10, and Z⁻ is an anion.

5. A use according to claim 1, wherein X'-Q is selected from 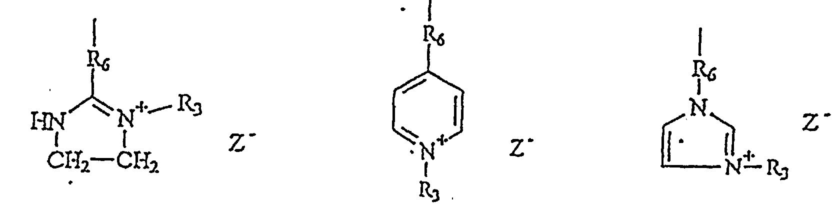 wherein R₆ is a single bond or an allcylene group of 1 to 6 carbon atoms, R₃ is a C₈ to C₂₅ hydrocarbyl group unsubstituted or substihited with at least one halo, amino, nitro, hydroxyl or aryl group, and Z⁻ is an anion.

6. A use according to claim 4 or 5, wherein X is selected from -Hal, -R₄-Hal, -O-CO-R₄-Hal, -CO-O-R₄-Hal, -CO-Hal, -CO-O-R₄-O-CO-R₄-Hal,
-C₆H₄-R₄-Hal; -CO-NH-O-CO-R₄-Hal; 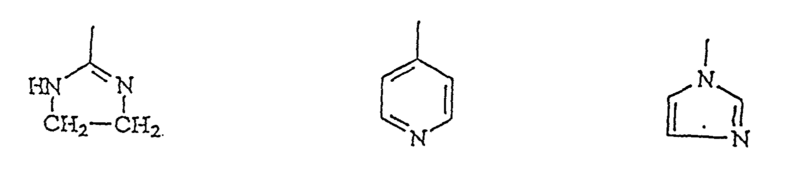 and Y is selected from -H, -OH, -CONH₂, -CO-O-R₄-OH, -R₄-OH, -CO-NH-R₄-OH, -C₆H₅, -C₆H₄-R₅, -CO-O-R₅, and -CO-O-R₄-O-R₄-OH;
wherein R₄ is an alkylene group of 1 to 6 carbon atoms, R₅ is an alkyl group of 1 to 6 carbon atoms and Hal is a halogen.

7. A use according to claim 6, wherein R₁ and R₂ are alkyl,
R₄ is methylene or ethylene, and Z⁻ is halide.

8. A use according to claim 7, wherein R₃ is a linear saturated hydrocarbyl group of 12 to 18 carbon atoms.

9. A use according to claim 6, wherein m and p are both integers.

10. A use according to claim 6, wherein n+m+p is from 200 to 1000.

11. A use according to claim 6 , wherein R is H and Y is H.

12. A use according to claim 4, wherein X'-Q is -O-CO-CH₂-Q,
X is -O-CO-CH₂-Cl, R is H and Y is H or OH.

13. A use according to claim 4, wherein Q is -N+R₁R₂R₃Z⁻.

14. A use according to claim 13, wherein R₁ and R₂ are alkyl, R₃ is a linear saturated hydrocarbyl group of 12 to 18 carbon atoms, and Z⁻ is halide.

15. A use according to claim 4, wherein X'-Q is -C₆H₄-CH₂-Q, X is -C₆H₄-CH₂-Cl, and p is 0.

16. A use according to claim 5, wherein X'-Q is 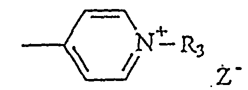 and X is 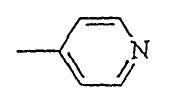 wherein R is H and Z⁻ is halide.

17. A topical antiperspirant composition for application to the skin comprising 1% to 20% by weight of a film-forming polymer as defined in claim 1, dissolved in a cosmetically acceptable non-aqueous solvent carrier.

18. The topical composition of claim 17, wherein the non-aqueous solvent carrier comprises ethanol, propylene glycol, butylene glycol, dipropylene glycol, glycerol, propylene carbonate, volatile silicone, or a mixture of two or more of these.

19. The topical composition of claim 17 or 18 wherein the non-aqueous solvent carrier comprises ethanol and volatile silicone.

20. The topical composition of claims 17 to 19, wherein the non-aqueous solvent carrier additionally comprises up to 30% water, wherein the amount of water is sufficiently low to maintain the composition as a clear solution or stable dispersion.

## Patentansprüche

1. Verwendung, nichtherapeutisch, einer Lösung oder Dispersion eines filmbildenden Polymers als ein Antihydrotikum, welches Polymer die Einheiten mit der Formel aufweist: 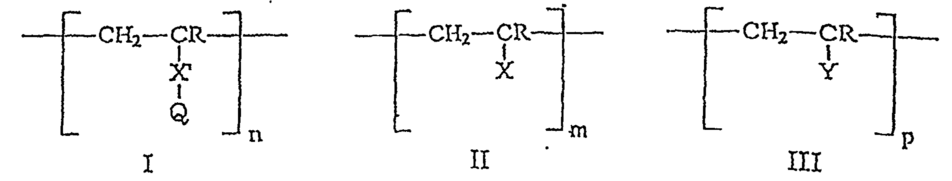 worin sind:
X eine Gruppen, die durch Reaktion mit einem Amin direkt quaternisierl werden kann oder selbst ein quaternisierbares Stickstoffatom aufweist;
X'-Q quaternisiertes X, worin Q die quaternisierte Stickstoff-Gruppe isl und X' nicht vorhanden zu sein braucht;
Q hat mindestens ein quaternisiertes Stickstoffatom mit mindestens einem Substituenten daran, der hydrophob ist und mindestens 8 Kohlenstoffatome enthält;
Y ein beliebiges Atom oder eine beliebige Gruppe, die nicht direkt quaternisiert werden können und selbst kein quaternisiertes Stickstoffatom aufweisen;
R Wasserstoff oder Alkyl;
m und p können unabhängig Null oder eine ganze Zahl sein;
(n+m+p) 20 bis 200;
n/(n+m+p) in Prozent ausgedrückt von 1% bis 100%;
und worin das Polymer wahlweise zwei oder mehrere Einheiten enthalten kann, die von Formel I, Formel II und/oder Formel III verschieden sind.

2. Verwendung nach Anspruch 1, wobei n/(n+m+p)25 bis 95% beträgt.

3. Verwendung nach Anspruch 1, wobei m und p beides ganze Zahlen sind und n/(n+m+p) 1% oder weniger als 100% beträgt.

4. Verwendung nach Anspruch 1, wobei X'-Q ausgewählt ist aus:
-CO-O-R₄-Q -O-CO-R₄-Q -CO-Q
R₄-CO-O-Q C₆H₄-R₄-Q
-CO-O-R₄-O-CO-R₄-Q -R₄-O-CO-R₄-Q -CO-NH-CH₂-O-CO-R₄-Q
worin jedes R₄ unabhängig eine Alkyl-Gruppe mit 1 bis 6 Kohlenstoffatomen ist und Q ausgewählt wird aus:
(a) -N⁺R₁R₂R₃ Z⁻ 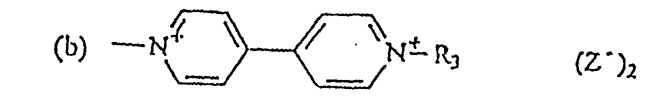 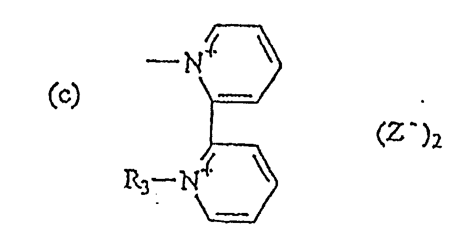 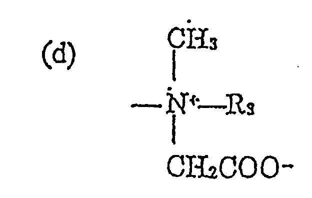 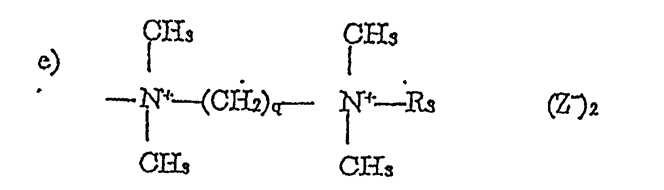
worin R₁ und R₂ gleich oder verschieden sind und jedes eine C₁- bis C₈-Hydrocarbyl-Gruppe ist, unsubstituiert oder substituiert mit einer Halogen-, Amino-, Nitro-, Hyroxyl- oder Aryl-Gruppe; R₃ ist eine C₈- bis C₂₅-Hydrocarbyl-Gruppe, unsubstituiert oder substituiert mit einer Halogen-, Amino-, Nitro-, Hyroxyl- oder Aryl-Gruppe; q ist eine ganze Zahl von 2 bis 10 und Z⁻ ist ein Anion.

5. Verwendung nach Anspruch 1, worin X'-Q ausgewählt ist aus : 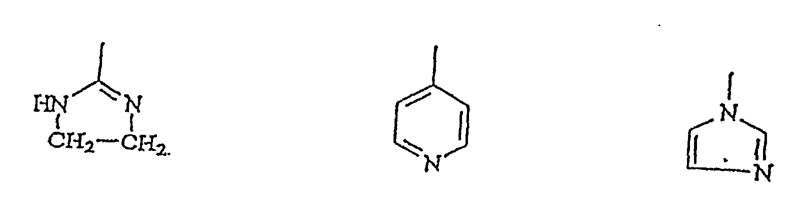 worin R₆ eine Einfachbindung oder eine Alkylen-Gruppe mit 1 bis 6 Kohlenstoffatomen ist, R₃ ist eine C₈- bis C₂₅-Hydrocarbyl-Gruppe, unsubstituiert oder substituiert mit einer Halogen-, Amino-, Nitro-, Hyroxyl- oder Aryl-Gruppe; und Z⁻ ist ein Anion.

6. Verwendung nach Anspruch 4 oder 5, worin X ausgewählt ist aus: -Hal, -R₄-Hal, -O-CO-R₄-Hal, -CO-O-R₄-Hal, -CO-Hal, -CO-O-R₄-O-CO-R₄-Hal, -C₆R₄Hal, -CO-NH-O-CO-R₄-Hal; 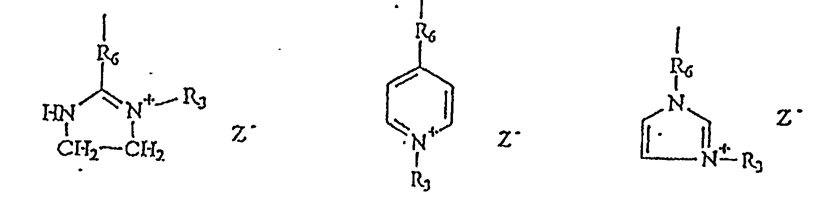 und Y ausgewählt aus: -H, -OH, -CONH₂, -CO-O-R₄-OH, -R₄-OH,
-CO-NH-R₄-OH, -C₆H₅, -C₆H₄R₅, -CO-O-R₅ und -CO-O-R₄-O-R₄-OH;
worin R₄ eine Alkylen-Gruppe ist 1 bis 6 Kohlenstoffatomen ist, R₅ ist eine Alkyl-Gruppe mit 1 bis 6 Kohlenstoffatomen, und Hal ist ein Halogen.

7. Verwendung nach Anspruch 6, worin R₁ und R₂ Alkyl sind, R₄ ist Methylen oder Ethylen und Z⁻ ist Halogenid.

8. Verwendung nach Anspruch 7, worin R₃ eine lineare, gesättigte Hydrocarbyl-Gruppe mit 12 bis 18 Kohlenstoffatomen ist.

9. Verwendung nach Anspruch 6, worin m und p beides ganze Zahlen sind.

10. Verwendung nach Anspruch 6, worin m+n+p 200 bis 1.000 betragen.

11. Verwendung nach Anspruch 6, worin R H ist und Y H ist.

12. Verwendung nach Anspruch 4, worin X'-Q ist -O-CO-CH₂-Q, X ist -O-CO-CH₂-Cl, R ist H und Y ist H oder OH.

13. Verwendung nach Anspruch 4, worin Q ist N⁺R₁R₂R₃Z⁻.

14. Verwendung nach Anspruch 13, worin R₁ und R₂ Alkyl sind, R₃ ist eine lineare, gesättigte Hydrocarbyl-Gruppe mit 12 bis 18 Kohlenstoffatomen und Z⁻ ist ein Halogenid.

15. Verwendung nach Anspruch 4, worin X'-Q ist -C₆H₄-CH₂-Q, X ist
-C₆H₄-CH₂-Cl und p ist Null.

16. Verwendung nach Anspruch 5, worin X'-Q ist 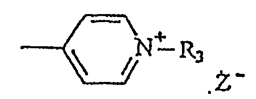 und X ist 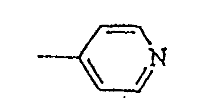 worin R H ist und Z⁻ Halogenid ist.

17. Topische antihydrotische Zusammensetzung zur Aufbringung auf die Haut, aufweisend 1% bis 20 Gewichtsprozent eines filmbildenden Polymers nach Anspruch 1, aufgelöst in einem kosmetisch annehmbaren, nichtwässrigen Lösemittel-Träger.

18. Topische Zusammensetzung nach Anspruch 17, worin der nichtwässrigen Lösemittel-Träger aufweist: Ethanol, Propylenglykol, Butylenglykol, Dipropylenglykol, Glycerin, Propylencarbonat, leichtverdampfendes Silicon oder eine Mischung von zwei oder mehreren davon.

19. Topische Zusammensetzung nach Anspruch 17 oder 18, worin der nichtwässrige Lösemittel-Träger Ethanol und leichtverdampfendes Silicon aufweist.

20. Topische Zusammensetzung nach Anspruch 17 bis 19, worin der nichtwässrige Lösemittel-Träger zusätzlich bis zu 30% Wasser aufweist, wobei die Wassermenge ausreichend gering ist, um die Zusammensetzung als eine klare Lösung oder stabile Dispersion zu bewahren.

## Revendications

1. Utilisation non thérapeutique, en tant qu'antitranspirant, d'une solution ou d'une dispersion d'un polymère filmogène comprenant des motifs de formules: 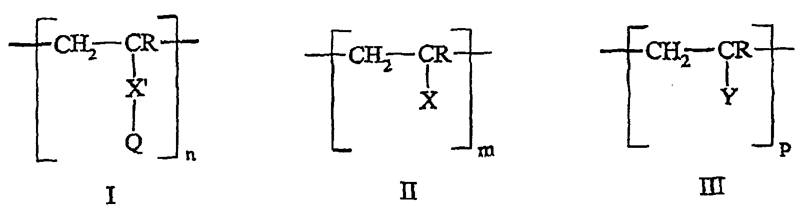 dans lesquelles:
X est un groupe, qui peut être directement quaternisé par réaction avec une amine ou qui comprend lui-même un atome d'azote qui peut être quatemisé;
X'-Q est un groupe X quaternisé, où Q est le groupe azote quatemisé et X' peut ne pas exister,
Q comporte au moins un atome d'azote quaternisé sur lequel est présent au moins un substituant qui est hydrophobe et qui contient au moins 8 atomes de carbone;
Y est n'importe quel atome ou groupe, qui ne peut pas être directement quatemisé et qui ne comprend pas lui-même un atome d'azote qui peut être quaternisé;
R est un atome d'hydrogène ou un groupe alkyle;
m et p peuvent être indépendamment 0 ou un entier;
(n + m + p) est compris entre 20 et 2000;
n/(n + m + p) exprimé en pourcentage est compris entre 1% et 100%;
et dans laquelle le polymère peut éventuellement contenir deux motifs différents ou plus de formule I, de formule II et/ou de formule III.

2. Utilisation selon la revendication 1, dans laquelle n/(n + m + p)% est compris entre 25% et 95%.

3. Utilisation selon la revendication 1, dans laquelle m et p sont tous deux des entiers et n/(n + m + p)% est compris entre 1% et moins de 100%.

4. Utilisation selon la revendication 1, dans laquelle X'-Q est choisi parmi les groupes
-CO-O-R₄-Q -O-CO-R₄-Q -CO-Q
-R₄-CO-O-Q -C₆H₄-R₄-Q -CO-O-R₄-O-CO-R₄-Q
-R₄-O-CO-R₄-Q -CO-NH-CH₂O-CO-R₄-Q -CO-O-R₄-O-R₄-Q
-CO-O-R₄-O-R₄-O-CO-R₄-Q
dans lesquels chaque R₄ est indépendamment un groupe alkylène ayant 1 à 6 atomes de carbone et
Q est choisi parmi les groupes
(a) ―N⁺R₁R₂R₃ Z⁻ 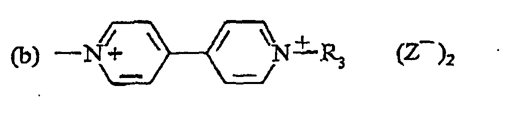 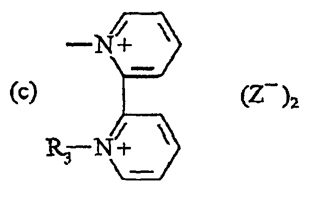 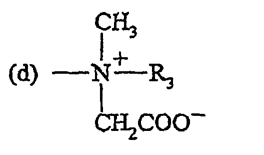 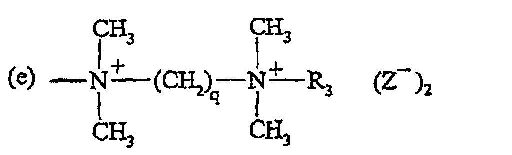
dans lesquels R₁ et R₂ sont identiques ou différents et sont chacun un groupe hydrocarbyle en C₁ à C₈ non substitué ou substitué par au moins un groupe halogène, amino, nitro, hydroxy ou aryle, R₃ est un groupe hydrocarbyle en C₈ à C₂₅ non substitué ou substitué par au moins un groupe halogène, amino, nitro, hydroxy ou aryle, q est un entier compris entre 2 et 10 et Z⁻ est un anion.

5. Utilisation selon la revendication 1, dans laquelle X'-Q est choisi parmi les groupes 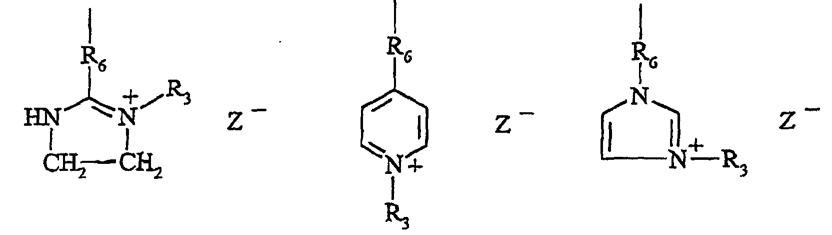 dans lesquels R₆ est une simple liaison ou un groupe alkylène ayant 1 à 6 atomes de carbone, R₃ est un groupe hydrocarbyle en C₈ à C₂₅ non substitué ou substitué par au moins un groupe halogène, amino, nitro, hydroxy ou aryle et Z est un anion.

6. Utilisation selon la revendication 4 ou 5, dans laquelle X est choisi parmi -Hal, -R₄-Hal, -O-CO-R₄-Hal, -CO-O-R₄-Hal, -CO-Hal,
-CO-O-R₄-O-CO-R₄-Hal, -C₆H₄-R₄-Hal, -CO-NH-O-CO-R₄-Hal, 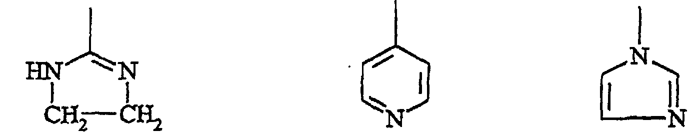 et Y est choisi parmi les groupes -H, -OH, -CONH₂, -CO-O-R₄-OH, -R₄-OH,
-CO-NH-R₄-OH, -C₆H₅, -C₆H₄-R₅, -CO-O-R₅ et -CO-O-R₄-O-R₄-OH;
dans lesquels R₄ est un groupe alkylène ayant 1 à 6 atomes de carbone, R₅ est un groupe alkyle ayant 1 à 6 atomes de carbone et Hal est un atome d'halogène.

7. Utilisation selon la revendication 6, dans laquelle R₁ et R₂ sont un groupe alkyle, R₄ est un groupe méthylène ou éthylène et Z⁻ est un halogénure.

8. Utilisation selon la revendication 7, dans laquelle R₃ est un groupe hydrocarbyle linéaire saturé ayant 12 à 18 atomes de carbone.

9. Utilisation selon la revendication 6, dans laquelle m et p sont tous deux des entiers.

10. Utilisation selon la revendication 6, dans laquelle n + m + p est compris entre 200 et 1000.

11. Utilisation selon la revendication 6, dans laquelle R est H et Y est H.

12. Utilisation selon la revendication 4, dans laquelle X'-Q est un groupe -O-CO-CH₂-Q, X est un groupe -O-CO-CH₂-Cl, R est H et Y est H ou OH.

13. Utilisation selon la revendication 4, dans laquelle Q est -N+R₁R₂R₃Z⁻.

14. Utilisation selon la revendication 13, dans laquelle R₁ et R₂ sont un groupe alkyle, R₃ est un groupe hydrocarbyle linéaire saturé ayant 12 à 18 atomes de carbone et Z⁻ est un halogénure.

15. Utilisation selon la revendication 4, dans laquelle X'-Q est un groupe -C₆H₄-CH₂-Q, X est un groupe -C₆H₄-CH₂-Cl et p est 0.

16. Utilisation selon la revendication 5, dans laquelle X'-Q est un groupe 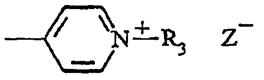 et X est un groupe 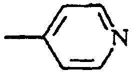 dans lequel R est H et Z est un halogénure.

17. Composition topique antitranspirante pour une application sur la peau comprenant 1% à 20% en masse d'un polymère filmogène tel que défini dans la revendication 1, dissous dans un excipient constitué par un solvant non aqueux acceptable du point de vue cosmétique.

18. Composition topique selon la revendication 17, dans laquelle l'excipient constitué par un solvant non aqueux comprend l'éthanol, le propylèneglycol, le butylèneglycol, le dipropylèneglycol, le glycérol, le carbonate de propylène, un silicone volatil ou un mélange d'au moins deux de ces solvants.

19. Composition topique selon la revendication 17 ou 18, dans laquelle l'excipient constitué par un solvant non aqueux comprend l'éthanol et un silicone volatil.

20. Composition topique selon les revendications 17 à 19, dans laquelle l'excipient constitué par un solvant non aqueux comprend en outre jusqu'à 30% d'eau, où la quantité d'eau est suffisamment faible pour maintenir la composition sous forme d'une solution limpide ou d'une dispersion stable.
